# EUROPÄISCHE PATENTSCHRIFT

(11) **EP 1 831 179 B1**
(45) Veröffentlichungstag und Bekanntmachung des Hinweises auf die Patenterteilung: **03.11.2010**
(21) Anmeldenummer: 05807705.8
(22) Anmeldetag: 18.11.2005
(51) Int. Cl.: C07D 233/58, C07D 213/74, C07D 213/18, C07D 295/037, C07C 279/02, C07C 275/02, C07F 9/54

(54) **VERFAHREN ZUR HERSTELLUNG VON ONIUM-SALZEN MIT TETRAFLUOROBORAT-ANION MIT REDUZIERTEM HALOGENID-GEHALT**
METHOD FOR THE PRODUCTION OF ONIUM SALTS WITH TETRAFLUOROBORATE ANION HAVING A REDUCED HALIDE CONTENT
PROCEDE DE FABRICATION DE SELS D'ONIUM CONTENANT DES ANIONS DE TETRAFLUOROBORATE PRESENTANT UNE TENEUR REDUITE EN HALOGENURES

(30) Priorität: 14.12.2004 DE 102004060073; 27.07.2005 DE 102005035103
(43) Veröffentlichungstag der Anmeldung: 12.09.2007
(73) Patentinhaber: Merck Patent GmbH, 64293 Darmstadt (DE)
(72) Erfinder: IGNATYEV, Nikolai, 47058 Duisburg (DE); WELZ-BIERMANN, Urs, 64646 Heppenheim (DE); KUCHERYNA, Andriy, 42117 Wuppertal (DE); WILLNER, Helge, 45481 Muelheim/Ruhr (DE)
(86) Internationale Anmeldenummer: PCT/EP2005/012398
(87) Internationale Veröffentlichungsnummer: WO 2006/063653

(56) Entgegenhaltungen:
- EP-A- 1 182 197
- US-A1- 2003 080 312
- US-B1- 6 245 918
- HOLDERBERG A W ET AL: "Electrophile Induced Addition Reactions of Bis-phosphonio-isophosphin dolide Cations" TETRAHEDRON, ELSEVIER SCIENCE PUBLISHERS, AMSTERDAM, NL, Bd. 56, Nr. 1, Januar 2000 (2000-01), Seiten 57-62, XP004186965 ISSN: 0040-4020

## Beschreibung

Die Erfindung betrifft ein Verfahren zur Herstellung von Onium-Salzen mit Tetrafluoroborat-Anion durch Umsetzung eines Onium-Halogenids mit einem Oxonium-Tetrafluoroborat, Sulfonium-Tetrafluoroborat oder Triphenylcarbonium-Tetrafluoroborat.

Eine große Anzahl von Onium-Salzen sind ionische Flüssigkeiten. Ionische Flüssigkeiten stellen durch Ihre Eigenschaften in der modernen Forschung eine wirksame Alternative zu traditionellen flüchtigen organischen Solventien für die organische Synthese dar. Die Verwendung von ionischen Flüssigkeiten als neues Reaktionsmedium könnte weiterhin eine praktische Lösung sowohl für die Lösungsmittel Emission als auch für Probleme in der Wiederaufbereitung von Katalysatoren sein (R. Sheldon "Catalytic reactions in ionic liquids", Chem. Commun., 2001, 2399-2407; M.J. Earle, K.R. Seddon "Ionic liquids. Green solvent for the future", Pure Appl. Chem., 72 (2000), 1391-1398; P. Wasserscheid, W. Keim "Ionische Flüssigkeiten - neue Lösungen für die Übergangsmetallkatalyse", Angew. Chem., 112 (2000), 3926-3945; T. Welton "Room temperature ionic liquids. Solvents for synthesis and catalysis", Chem. Rev., 92 (1999), 2071-2083 oder R. Hagiwara, Ya. Ito "Room temperature ionic liquids of alkylimidazolium cations and fluoroanions", J. Fluorine Chem., 105 (2000), 221-227).

Ionische Flüssigkeiten oder flüssige Salze sind ionische Spezies, die aus einem organischen Kation und einem in der Regel anorganischen Anion bestehen. Sie enthalten keine neutralen Moleküle und weisen meistens Schmelzpunkte kleiner 373 K auf. Der Schmelzpunkt kann jedoch auch höher liegen, ohne die Anwendbarkeit der Salze auf allen Anwendungsgebieten zu beschränken. Beispiele für organische Kationen sind unter anderem Tetraalkylammonium, Tetraalkylphosphonium, N-Alkylpyridinium, 1,3-Dialkylimidazolium oder Trialkylsulfonium. Unter einer Vielzahl von geeigneten Anionen sind beispielsweise BF₄⁻, PF₆⁻, SbF₆⁻, NO₃⁻, CF₃SO₃⁻, (CF₃SO₂)₂N⁻, ArylSO₃⁻, CF₃CO₂⁻, CH₃CO₂⁻ oder Al₂Cl₇⁻ zu nennen.

Die Eigenschaften der ionischen Flüssigkeiten, beispielsweise der Schmelzpunkt, die thermische und elektrochemische Stabilität oder Viskosität, werden durch die Auswahl der Kationen und Anionen bestimmt. Ionische Flüssigkeiten sind nicht flüchtige Materialien und können daher nicht durch herkömmliche Methoden der Aufreinigung, wie beispielsweise der Destillation, gereinigt werden, weil sie für die meisten organischen Lösungsmittel entwickelt wurden.

Daher ist bei Verfahren zur Herstellung von Onium-Salzen, insbesondere ionischer Flüssigkeiten mit Tetrafluoroborat-Anion, die Technologie von entscheidender Bedeutung, damit diese durch die Reaktion per se oder die Reaktionsführung mit geringen Verunreinigungen synthetisiert werden können. Eine vorwiegend vorhandene Verunreinigung in bekannten ionischen Flüssigkeiten sind Halogenid-Ionen. Ist der Anteil an Halogenid-Ionen, beispielsweise Chlorid-Ionen, größer als 1000 ppm (0,1 %), so reduziert sich die Anwendbarkeit der ionischen Flüssigkeit, insbesondere in der Anwendung für elektrochemische Prozesse.

Holderberg et al. ("Electrophile induced addition reactions of bisphosphonio-isophosphin dolide cations", Tetrahedron 56, 2000, 57-62) offenbart die Metathese-Reaktion von Phosphoniumbromid mit Meerweinsalz [Me₃O][BF_{4]}, in der Br⁻ durch [BF₄]⁻ ausgetauscht wird.
EP 1182197 A1 beschreibt die Lösungsmittel-freie Eintopfreaktion von 1-Methylimidazol mit einem Alkylchlorid und Natriumtetrafluoroborat, bei der Imidazoliumtetrafluoroborate erhalten werden.
In einem weiteren Dokument (US 2003/080312 A1) wird die Synthese von Halogenid-freien Ionischen Flüssigkeiten durch die Umsetzung von N-Methylimidazol mit Trifluoressigsäureethylester beschrieben. Nach einem Anionenaustausch mit HBF₄ erhält man neben Trifluoressigsäure das Tetrafluoroborat-Salz.
Eine weitere Möglichkeit zur Synthese von Halogenid-freien Ionischen Flüssigkeiten aus N-Methylimidazol und Meerweinsalz wird in US B1 6245918 offenbart.

Aufgabe der vorliegenden Erfindung war dementsprechend ein alternatives Verfahren zur Herstellung von Onium-Tetrafluoroboraten mit geringem Chlorid-Gehalt zur Verfügung zu stellen, welches zu Produkten mit hoher Reinheit in guter Ausbeute führt und auch für eine großtechnische Produktion geeignet ist.

Die Aufgabe wird durch das erfindungsgemäße Verfahren gelöst. Gegenstand der Erfindung ist demgemäss ein Verfahren zur Herstellung von Onium-Tetrafluoroboraten durch Umsetzung eines Onium-Halogenids mit einem Trialkyloxonium-Tetrafluoroborat, Sulfonium-Tetrafluoroborat oder Triphenylcarbonium-Tetrafluoroborat.

Das erfindungsgemäße Verfahren ist eine Verbesserung der bekannten Syntheseverfahren für Onium-Tetrafluoroborate, die in der Regel 2-StufenVerfahren sind, wie in P. Wasserscheid und W. Keim, Angew. Chem. 112 (2000), 3926-3945 beschrieben. Im ersten Schritt der bekannten Verfahren wird eine organische Base, typischerweise ein Amin, Phosphin oder eine heterocyclische Verbindung, mit einem Alkylhalogenid alkyliert und das entstehende Onium-Halogenid im zweiten Schritt über einen Anionenaustausch in das Tetrafluoroborat umgewandelt.

In der zweiten Stufe wird das Halogenid, beispielsweise 1-Ethyl-3-methylimidazoliumchlorid oder -bromid, mit NaBF₄ in Aceton nach S. Park und R. J. Kazlauskas, J. Organic Chemistry, 66 (2001), 8395-8401, mit NaBF₄ in Wasser nach R. Karmakar und A. Samanta, J. Phys. Chem. A, 106 (2002), 6670-6675, mit AgBF₄ oder HBF₄ in Wasser nach J. D. Holbrey und K. R. Seddon, J. Chem. Soc., Dalton Trans., (1999), 2133-2139, mit NH₄BF₄ in Aceton nach J. Fuller et al, J. Electrochem. Soc., 144 (1997), 3881-3885, mit HBF₄ in Methanol nach T. Nishida et al, J. of Fluorine Chem., 120 (2003), 135-141 oder mit NH₄BF₄ unter Mikrowellenbestrahlung nach V.V. Namboodiri und R. S. Varma, Tetrahedron Lett., 43 (2002), 5381-5383 umgesetzt.

Alle bekannten Verfahren haben einen Nachteil, insbesondere für eine großtechnische Synthese. Silbertetrafluoroborat ist beispielsweise ein teures Reagenz. Die Umsetzungen mit NaBF₄, NH₄BF₄ und HBF₄ in Wasser erfordern einen Aufreinigungsschritt, eventuell durch Verwendung von AgBF₄ oder Adsorbentien. HBF₄ in Methanol ist nicht kommerziell erhältlich und ist teurer als wässrige HBF₄, die wiederum kommerziell erhältlich ist. Bei der Umsetzung in wässriger HBF₄ entsteht jedoch die Halogenwasserstoffsäure als Nebenprodukt, die aus dem Endprodukt nicht durch Destillation entfernt werden kann, da sich im Wasser zwei Salze und zwei Säuren im Gleichgewicht befinden. Die erhaltenen Onium-Tetrafluoroborate enthalten zwangsläufig immer einige Prozente an Halogenidionen, dokumentiert durch Untersuchungen von N.M.M Mateus et al, Green Chemistry, 5 (2003), 347-352.

Überraschenderweise wurde ein einfaches Verfahren entwickelt. Bei der Umsetzung eines Onium-Halogenids, beispielsweise einem Chlorid, Bromid oder Iodid, mit einem Oxonium Tetrafluoroborat, beispielsweise Meerwein Salz, mit einem Sulfonium-Tetrafluoroborat oder Triphenylcarbonium Tetrafluoroborat, entstehen daher Onium-Tetrafluoroborate und Alkylhalogenide oder Triphenylhalogenide und Dialkylether oder Dialkylsulfide als Nebenprodukte, die entweder Gase oder leicht flüchtige Verbindungen sind und ohne großen prozesstechnischen Aufwand aus der Reaktionsmischung entfernt werden können. Einige dieser Nebenprodukte sind selbst wertvolle Materialien für organische Synthesen.

Das erfindungsgemäße Verfahren erlaubt die Synthese einer Vielzahl von Tetrafluoroborat-Salzen, wobei unterschiedliche Substituenten, beispielsweise Alkylgruppen, am Onium-Kation vorliegen können, sogenannte unsymmetrische Verbindungen. Die neue Methode kann jedoch auch zur Reinigung von Tetrafluoroboraten verwendet werden, die Chlorid-, Bromid- oder Iodid-Anionen als Verunreinigungen enthalten. Man erhält so ionische Flüssigkeiten mit Tetrafluoroborat-Anionen in hoher Qualität ohne den Einsatz von teuren Materialien wie Silbertetrafluoroborat bzw. ohne Verunreinigungen an Silberkationen.

Geeignete Onium-Halogenide nach Anspruch 1 sind bei Umsetzung mit Trialkyloxonium-Tetrafluoroborat oder Triphenylcarbonium-Tetrafluoroborat Phosphoniumhalogenide, Thiouroniumhalogenide, Guanidiniumhalogenide oder Halogenide mit heterocyclischem Kation oder bei Umsetzung mit Trialkylsulfonium-Tetrafluoroborat Ammoniumhalogenide, Phosphoniumhalogenide, Thiouroniumhalogenide, Guanidiniumhalogenide oder Halogenide mit heterocyclischem Kation, wobei die Halogenide aus der Gruppe Chloride, Bromide oder Iodide ausgewählt werden können. Bevorzugt werden im erfindungsgemäßen Verfahren Chloride oder Bromide eingesetzt. Im erfindungsgemäßen Verfahren werden für die Klasse der Thiouronium-Salze bevorzugt Thiouroniumiodide eingesetzt.

Die Onium-Halogenide sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Folgende Onium-Halogenide nach Anspruch 1 werden für das erfindungsgemäße Verfahren eingesetzt.

Ammonium- und Phosphoniumhalogenide können beispielsweise durch die Formel (1)

[XR₄]⁺ Hal⁻ (1),

beschrieben werden,
wobei
X N, P
Hal Cl, Br oder I und
R jeweils unabhängig voneinander
H, wobei nicht alle Substituenten R gleichzeitig H sein dürfen, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen, gesättigtes, teilweise oder vollständig ungesättigtes Cycloalkyl mit 3-7 C-Atomen, das mit Alkylgruppen mit 1-6 C-Atomen substituiert sein kann bedeutet,
wobei ein oder mehrere R teilweise oder vollständig mit F substituiert sein können, wobei jedoch nicht alle vier oder drei R vollständig mit F substituiert sein dürfen,
und wobei ein oder zwei nicht benachbarte und nicht α- oder ω-ständige Kohlenstoffatome des R, durch Atome und/oder Atomgruppierungen ausgewählt aus der Gruppe -O-, -S-, -S(O)- oder-SO₂- ersetzt sein können.

Ausgeschlossen sind jedoch Verbindungen der Formeln (1), in denen alle vier oder drei Substituenten R vollständig mit Halogenen substituiert sind, beispielsweise Tris(trifluormethyl)methylammoniumchlorid, Tetra(trifluormethyl)ammoniumchlorid oder Tetra(nonafluorbutyl)-ammoniumchlorid, Tris(trifluormethyl)methylphosphoniumchlorid, Tetra(trifluormethyl)phosphoniumchlorid oder Tetra(nonafluorbutyl)phosphoniumchlorid.

Thiouroniumhalogenide können beispielsweise durch die Formel (2)

[(R¹R²N)-C(=SR⁷)(NR³R⁴)]⁺ Hal⁻ (2)

und Guanidiniumhalogenide können beispielsweise durch die Formel (3)

[C(NR¹R²)(NR³R⁴)(NR⁵R⁶)]⁺ Hal⁻ (3),

beschrieben werden,
wobei
Hal bei Formel (2) Br oder I bedeutet und bei Formel (3) Cl, Br oder I bedeutet, und
R¹ bis R⁷ jeweils unabhängig voneinander
Wasserstoff oder CN, wobei Wasserstoff für R⁷ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁷ teilweise oder vollständig mit F substituiert sein können, wobei jedoch nicht alle Substituenten an einem N-Atom vollständig mit F substituiert sein dürfen, wobei die Substituenten R¹ bis R⁷ paarweise durch Einfach- oder Doppelbindung miteinander verbunden sein können
und wobei ein oder zwei nicht benachbarte, nicht direkt am Heteroatom gebundene und nicht ω-ständige Kohlenstoffatome der Substituenten R¹ bis R⁶ durch Atome und/oder Atomgruppierungen ausgewählt aus der Gruppe -O-, -S-, -S(O)- oder -SO₂- ersetzt sein können.

Halogenide mit heterocyclischem Kation können beispielsweise durch die Formel (4)

[HetN]⁺ Hal⁻ (4)

beschrieben werden, wobei
Hal Cl, Br oder I und
HetN⁺ ein heterocyclisches Kation, ausgewählt aus der Gruppe 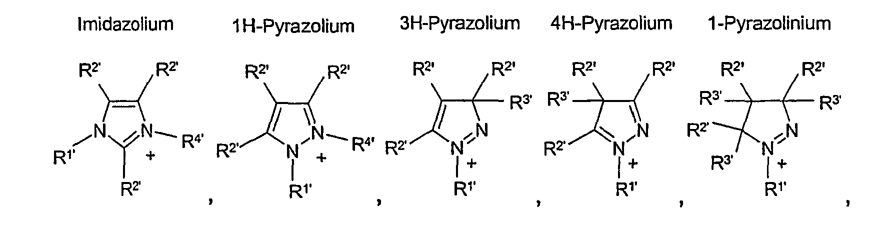 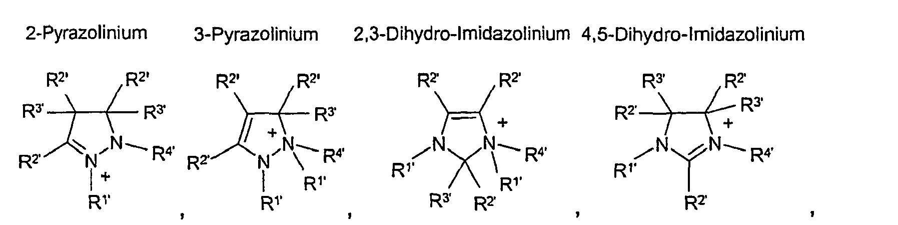 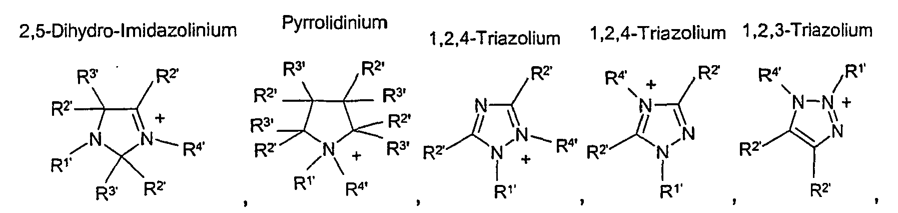 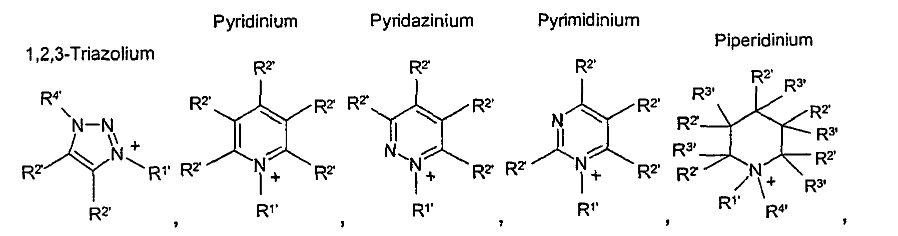 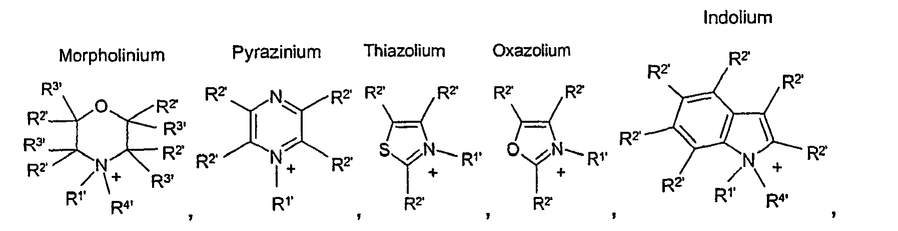 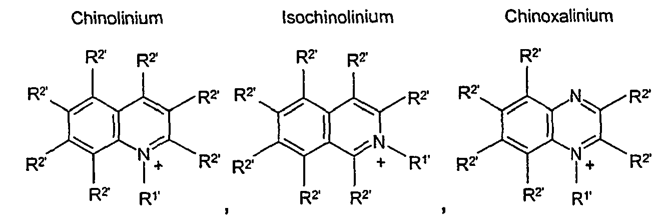 oder 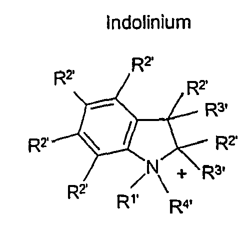 bedeutet, wobei die Substituenten
R¹' bis R⁴' jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7 C-Atomen, das mit Alkylgruppen mit 1-6 C-Atomen substituiert sein kann oder Aryl-C₁-C₆-alkyl bedeutet,
wobei die Substituenten R¹' und R⁴' teilweise oder vollständig mit F substituiert sein können, wobei jedoch nicht gleichzeitig R¹' und R⁴' CN sind oder vollständig mit F substituiert sein dürfen,
wobei die Substituenten R²' und R³' teilweise oder vollständig mit Halogenen oder teilweise mit NO₂ oder CN substituiert sein dürfen,
und wobei ein oder zwei nicht benachbarte, nicht direkt am Heteroatom gebundene und nicht ω-ständige Kohlenstoffatome der Substituenten R¹' bis R⁴', durch Atome und/oder Atomgruppierungen ausgewählt aus der Gruppe -O-, -S-, -S(O)- oder -SO₂- ersetzt sein können.

Unter vollständig ungesättigten Substituenten werden im Sinne der vorliegenden Erfindung auch aromatische Substituenten verstanden.

Als Substituenten R und R¹ bis R⁷ der Verbindungen der Formeln (1) bis (3) kommen erfindungsgemäß dabei neben Wasserstoff bevorzugt in Frage: C₁- bis C₂₀-, insbesondere C₁- bis C₁₄-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl.
Die Substituenten R und R¹ bis R⁷ können jedoch ebenfalls durch weitere funktionelle Gruppen substituiert sein, beispielsweise durch CN, SO₂R', SO₂OR' oder COOR'. R' bedeutet nicht, teilweise oder perfluoriertes C₁- bis C₆ Alkyl, C₃- bis C₇-Cycloalkyl, unsubstituiertes oder substituiertes Phenyl.

Die Substituenten R in den Verbindungen der Formel (1) können dabei gleich oder verschieden sein. Bevorzugt sind drei Substituenten in Formel (1) gleich und ein Substituent verschieden.

Der Substituent R ist insbesondere bevorzugt Methyl, Ethyl, Isopropyl, Propyl, Butyl, sek.-Butyl, Pentyl, Hexyl, Octyl, Decyl oder Tetradecyl.

Bis zu vier Substituenten des Guanidinium-Kations können auch paarweise derart verbunden sein, dass mono-, bi- oder polycyclische Kationen entstehen.
Ohne Einschränkung der Allgemeinheit sind Beispiele für solche Guanidinium-Kationen: 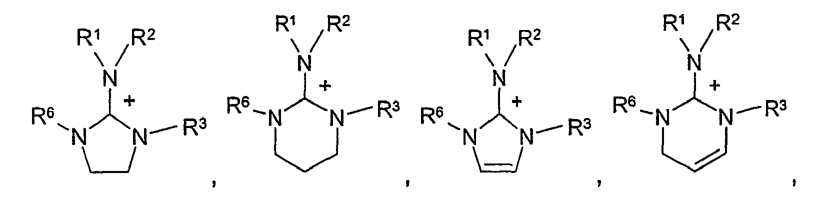 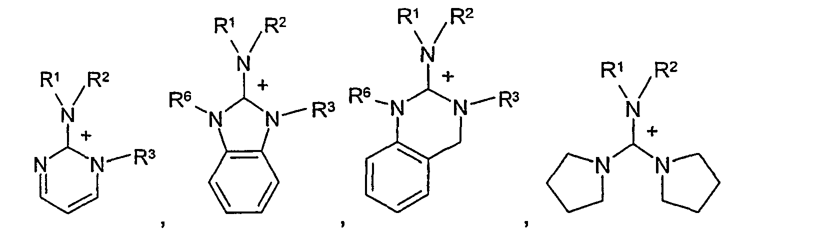 oder 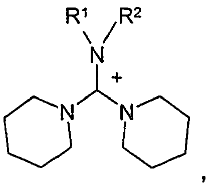 wobei die Substituenten R¹ bis R³ und R⁶ eine zuvor angegebene oder besonders bevorzugte Bedeutung haben können.

Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, C₁-C₆-Alkoxy, SCF₃, SO₂CH₃, SO₂CF₃, COOR", SO₂NR"₂, SO₂X', SO₃R", substituiertes oder unsubstituiertes Phenyl substituiert sein, wobei X' und R" eine zuvor oder später angegebene Bedeutung haben.

Bis zu vier Substituenten des des Thiouroniumkations [(R¹R²N)-C(=SR⁷)(NR³R⁴)]⁺ können auch paarweise derart verbunden sein, dass mono-, bi- oder polycyclische Kationen entstehen.
Ohne Einschränkung der Allgemeinheit sind Beispiele für solche Kationen im folgenden angegeben: 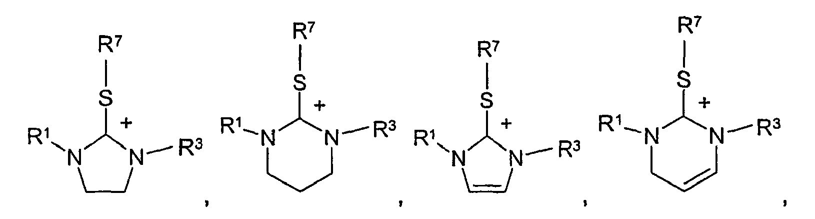 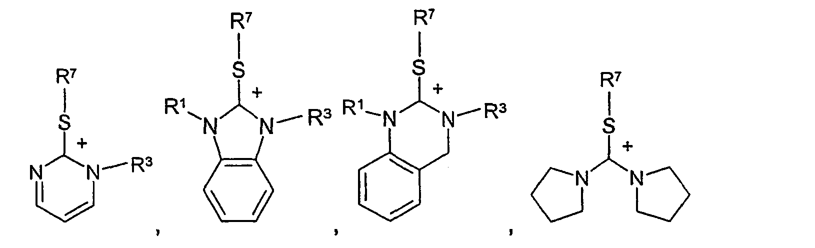 oder 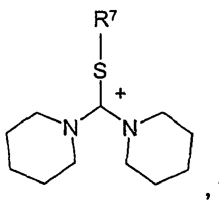 wobei die Substituenten R¹, R³ und R⁷ eine zuvor angegebene oder besonders bevorzugte Bedeutung haben können. Gegebenenfalls können die Carbocyclen oder Heterocyclen der zuvor angegebenen Guanidinium-Kationen noch durch C₁- bis C₆-Alkyl, C₁- bis C₆-Alkenyl, NO₂, F, Cl, Br, I, C₁-C₆-Alkoxy, SCF₃, SO₂CH₃, SO₂CF₃, COOR", SO₂NR"₂, SO₂X', SO₃R", substituiertes oder unsubstituiertes Phenyl substituiert sein, wobei X' und R" eine zuvor oder später angegebene Bedeutung haben.

Die C₁-C₁₄-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl oder Tetradecyl. Gegebenenfalls perfluoriert, beispielsweise als Difluormethyl, Trifluormethyl, Pentafluorethyl, Heptafluorpropyl oder Nonafluorbutyl.

Ein geradkettiges oder verzweigtes Alkenyl mit 2 bis 20 C-Atomen, wobei auch mehrere Doppelbindungen vorhanden sein können, ist beispielsweise Vinyl, Allyl, 2- oder 3-Butenyl, Isobutenyl, sek.-Butenyl, ferner 4-Pentenyl, iso-Pentenyl, Hexenyl, Heptenyl, Octenyl, -C₉H₁₇, -C₁₀H₁₉ bis -C₂₀H₃₉; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, -C₉H₁₅, -C₁₀H₁₇ bis -C₂₀H₃₇, vorzugsweise Ethinyl, 1- oder 2-Propinyl, 2- oder 3-Butinyl, 4-Pentinyl, 3-Pentinyl oder Hexinyl.

Aryl-C₁-C₆-alkyl bedeutet beispielsweise Benzyl, Phenylethyl, Phenylpropyl, Phenylbutyl, Phenylpentyl oder Phenylhexyl, wobei sowohl der Phenylring als auch die Alkylenkette, wie zuvor beschrieben teilweise oder vollständig mit F substituiert sein können, besonders bevorzugt Benzyl oder Phenylpropyl.

Der Phenylring oder auch die Alkylenkette können jedoch ebenfalls durch weitere funktionelle Gruppen substituiert sein, beispielsweise durch CN, SO₂R', SO₂OR' oder COOR'. R' hat dabei eine zuvor definierte Bedeutung.

Unsubstituierte gesättigte oder teilweise oder vollständig ungesättigte Cycloalkylgruppen mit 3-7 C-Atomen sind daher Cyclopropyl, Cyclobutyl, Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit C₁- bis C₆-Alkylgruppen substituiert sein können, wobei wiederum die Cycloalkylgruppe oder die mit C₁- bis C₆-Alkylgruppen substituierte Cycloalkylgruppe auch mit Halogenatomen wie F, Cl, Br oder I, insbesondere F oder Cl oder NO₂ substituiert sein kann. Die Cycloalkylgruppen können jedoch ebenfalls durch weitere funktionelle Gruppen substituiert sein, beispielsweise durch CN, SO₂R', SO₂OR' oder COOR'. R' hat dabei eine zuvor definierte Bedeutung.

In den Substituenten R, R¹ bis R⁶ oder R¹' bis R⁴' können auch ein oder zwei nicht benachbarte und nicht α-ständig zum Heteroatom oder ωgebundene Kohlenstoffatome, durch Atome und/oder Atomgruppierungen ausgewählt aus der Gruppe -O-, -S-, -S(O)- oder -SO₂- ersetzt sein.

Ohne Einschränkung der Allgemeinheit sind Beispiele für derart modifizierte Substituenten R, R¹ bis R⁶ und R¹' bis R⁴':
-OCH₃, -OCH(CH₃)₂, -CH₂OCH₃, -CH₂-CH₂-O-CH₃, -C₂H₄OCH(CH₃)₂, -C₂H₄SC₂H₅, -C₂H₄SCH(CH₃)₂, -S(O)CH₃, -SO₂CH₃, -SO₂C₆H₅, -SO₂C₃H₇, -SO₂CH(CH₃)₂, -SO₂CH₂CF₃, -CH₂SO₂CH₃, -O-C₄H₈-O-C₄H₉, -CF₃, -C₂F₅, -C₃F₇, -C₄F₉, -CF₂CF₂H, -CF₂CHFCF₃, -CF₂CH(CF₃)₂, -C₂F₄N(C₂F₅)C₂F₅, -CHF₂, -CH₂CF₃, -C₂F₂H₃, -C₃FH₆, -CH₂C₃F₇, -CH₂C(O)OCH₃, -CH₂C₆H₅ oder -C(O)C₆H₅.

In R' ist C₃- bis C₇-Cycloalkyl beispielweise Cyclopropyl, Cyclobutyl, Cyclopentyl, Cyclohexyl oder Cycloheptyl.

In R' bedeutet substituiertes Phenyl, durch C₁- bis C₆-Alkyl, C₁- bis C₆-Alkenyl, NO₂, F, Cl, Br, I, C₁-C₆-Alkoxy, SCF₃, SO₂CH₃, SO₂CF₃, COOR", SO₂X', SO₂NR"₂ oder SO₃R" substituiertes Phenyl, wobei X' F, Cl oder Br und R" ein nicht oder teilweise fluoriertes C₁- bis C₆-Alkyl oder C₃- bis C₇-Cycloalkyl wie für R' definiert bedeutet, beispielsweise, o-, m- oder p-Methylphenyl, o-, m- oder p-Ethylphenyl, o-, m- oder p-Propylphenyl, o-, m-oder p-Isopropylphenyl, o-, m- oder p-tert.-Butylphenyl, o-, m- oder p-Nitrophenyl, o-, m- oder p-Methoxyphenyl, o-, m- oder p-Ethoxyphenyl, o-, m-, p-(Trifluormethyl)phenyl, o-, m-, p-(Trifluormethoxy)phenyl, o-, m-, p-(Trifluormethylsulfonyl)phenyl, o-, m- oder p-Fluorphenyl, o-, m- oder p-Chlorphenyl, o-, m- oder p-Bromphenyl, o-, m- oder p-Iodphenyl, weiter bevorzugt 2,3-, 2,4-, 2,5-, 2,6-, 3,4- oder 3,5-Dimethylphenyl, 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,3-, 2,4-, 2,5-, 2,6-, 3,4- oder 3,5-Dimethoxyphenyl, 5-Fluor-2-methylphenyl, 3,4,5-Trimethoxyphenyl oder 2,4,5-Trimethylphenyl.

Die Substituenten R¹ bis R⁷ sind jeweils unabhängig voneinander bevorzugt eine geradkettige oder verzweigte Alkylgruppe mit 1 bis 10 C-Atomen. Die Substituenten R¹ und R², R³ und R⁴ und R⁵ und R⁶ in Verbindungen der Formeln (2) und (3) können dabei gleich oder verschieden sein. Besonders bevorzugt sind R¹ bis R⁷ jeweils unabhängig voneinander Methyl, Ethyl, n-Propyl, Isopropyl, n-Butyl, sek.-Butyl, Phenyl oder Cyclohexyl, ganz besonders bevorzugt Methyl, Ethyl, n-Propyl, Isopropyl oder n-Butyl.

Als Substituenten R¹' bis R⁴' von Verbindungen der Formel (4) kommen erfindungsgemäß dabei neben Wasserstoff bevorzugt in Frage: CN, C₁- bis C₂₀-, insbesondere C₁- bis C₁₂-Alkylgruppen, und gesättigte oder ungesättigte, d.h. auch aromatische, C₃- bis C₇-Cycloalkylgruppen, die mit C₁- bis C₆-Alkylgruppen substituiert sein können, insbesondere Phenyl oder Aryl-C₁-C₆-alkyl oder Diaminoalkyl mit C₁-C₄-Alkylgruppen, sofern diese nicht an das Heteroatom gebunden vorliegt.
Die Substituenten R¹' bis R⁴' können jedoch ebenfalls durch weitere funktionelle Gruppen substituiert sein, beispielsweise durch CN, SO₂R', SO₂OR' oder COOR'. R' bedeutet nicht, teilweise oder perfluoriertes C₁- bis C₆ Alkyl, C₃- bis C₇-Cycloalkyl, unsubstituiertes oder substituiertes Phenyl.

Die Substituenten R¹' und R⁴' sind jeweils unabhängig voneinander insbesondere bevorzugt CN, Methyl, Ethyl, Isopropyl, Propyl, Butyl, sek.-Butyl, Pentyl, Hexyl, Octyl, Decyl, Cyclohexyl, Phenyl, Phenylpropyl oder Benzyl. Sie sind ganz besonders bevorzugt CN, Methyl, Ethyl, n-Butyl oder Hexyl. In Pyrrolidinium-, Piperidinium oder Indolinium-Verbindungen sind die beiden Substituenten R¹' und R⁴' bevorzugt unterschiedlich.

Der Substituent R²' oder R³' ist jeweils unabhängig voneinander insbesondere Wasserstoff, Methyl, Ethyl, Isopropyl, Propyl, Butyl, sek.-Butyl, tert.-Butyl, Cyclohexyl, Dimethylamino, Diethylamino, Methylethylamino, Phenyl oder Benzyl. Besonders bevorzugt ist R²' Dimethylamino, Wasserstoff, Methyl, Ethyl, Isopropyl, Propyl, Butyl, sek.-Butyl oder tert.-Butyl. Ganz besonders bevorzugt sind R²' und R³' Wasserstoff, Dimethylamino oder Methyl.

HetN⁺ der Formel (4) ist bevorzugt 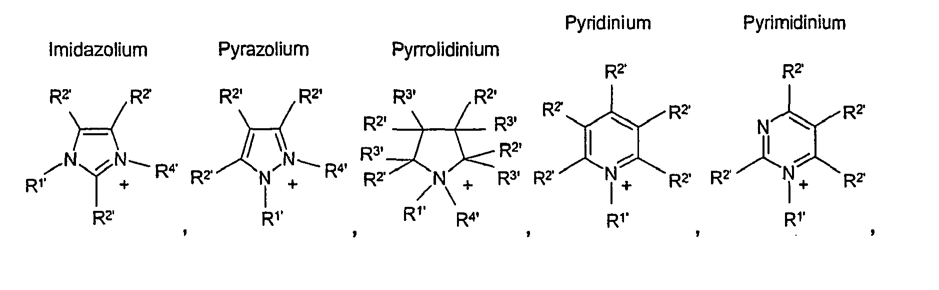 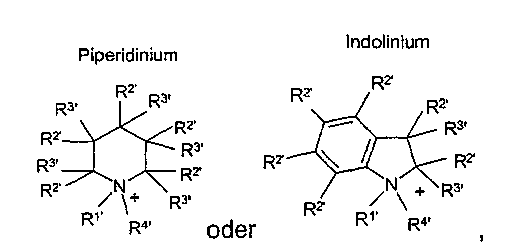 wobei die Substituenten R¹' bis R⁴' jeweils unabhängig voneinander eine zuvor beschriebene Bedeutung haben.

HetN⁺ ist besonders bevorzugt Imidazolium, Pyrrolidinium oder Pyridinium, wie zuvor definiert, wobei die Substituenten R¹' bis R⁴' jeweils unabhängig voneinander eine zuvor beschriebene Bedeutung haben.

Als Oxonium-Tetrafluoroborat mit der Formel [(Alkyl)₃O]⁺ [BF₄]⁻ wird bevorzugt ein Oxonium-Tetrafluoroborat mit geradkettigen oder verzweigten Alkylgruppen mit 1-8 C-Atomen, bevorzugt mit 1-4 C-Atomen eingesetzt, die jeweils unabhängig voneinander sind. Bevorzugt werden Oxonium-Tetrafluoroborate eingesetzt, bei denen die Alkylgruppen gleich sind. Verwendbar sind auch Tritylium Tetrafluoroborat, [(Phenyl)₃C]⁺ [BF₄]⁻.

Als Sulfonium-Tetrafluoroborat mit der Formel [(Alkyl)₃S]⁺ [BF₄]⁻ wird bevorzugt ein Sulfonium-Tetrafluoroborat mit geradkettigen oder verzweigten Alkylgruppen mit 1-8 C-Atomen, bevorzugt mit 1-4 C-Atomen eingesetzt, die jeweils unabhängig voneinander sind. Bevorzugt werden Sulfonium-Tetrafluoroborate eingesetzt, bei denen die Alkylgruppen gleich sind.

Die eingesetzten Oxonium-Tetrafluoroborate oder Sulfonium-Tetrafluoroborate sind in der Regel kommerziell erhältlich oder können nach Syntheseverfahren hergestellt werden, wie sie aus der Literatur, z.B. in den Standardwerken wie Houben-Weyl, Methoden der organischen Chemie, Georg-Thieme-Verlag, Stuttgart oder Richard C. Larock, Comprehensive Organic Transformations, 2nd Edition, Wiley-VCH, New York, 1999 bekannt sind. Dabei kann man auch von an sich bekannten, hier nicht näher erwähnten Varianten Gebrauch machen.

Beispiele für Oxonium-Tetrafluoroborate sind Trimethyloxonium-Tetrafluoroborat, Triethyloxonium-Tetrafluoroborat (Meerwein-Salz), Tris-(n-propyl)oxonium-Tetrafluoroborat, Dimethyl-ethyloxonium-Tetrafluoroborat, Diethyl-methyloxonium-Tetrafluoroborat oder Tris-(i-propyl)oxonium-Tetrafluoroborat. Ganz besonders bevorzugt wird Trimethyl- oder Triethyloxonium-Tetrafluoroborat eingesetzt.

Beispiele für Sulfonium-Tetrafluoroborate sind Trimethylsulfonium- , Triethylsulfonium- , Dimethylethylsulfonium-, Diethylmethylsulfonium-, Dipropylmethylsulfonium-, Dipropylethylsulfonium-, Dibutylmethylsulfonium-, Di-sec-butylmethylsulfonium-, Dibutylethylsulfonium-Tetrafluoroborat. Ganz besonders bevorzugt wird Trimethylsulfonium- und Triethylsulfonium-Tetrafluoroborat eingesetzt.

Ein allgemeines Schema fasst das erfindungsgemäße Verfahren zusammen:

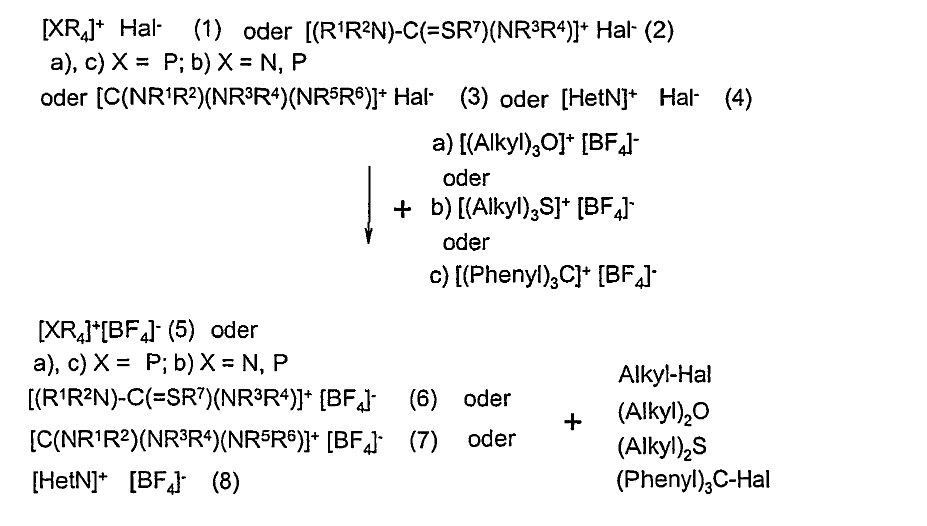

Die Substituenten R, R¹ bis R⁷ und HetN⁺ der Verbindungen der Formeln (1) bis (8) entsprechen den Bedeutungen, wie zuvor beschrieben.

Die Reaktion wird erfindungsgemäß bei Umsetzung mit Trialkyloxonium-Tetrafluoroborat oder Triphenylcarbonium-Tetrafluoroborat bei Temperaturen zwischen 0° und 100°C, bevorzugt bei 20° bis 50°, besonders bevorzugt bei Raumtemperatur durchgeführt. Bei Umsetzung mit Sulfonium-Tetrafluoroborat erfolgt die Reaktion erfindungsgemäß bei Temperaturen zwischen 0 und 150°C, bevorzugt bei 20 bis 100°C. Es wird kein Lösungsmittel benötigt. Es können jedoch auch Lösungsmittel eingesetzt werden, beispielsweise Dimethoxyethan, Acetonitril, Dichlormethan, Tetrahydrofuran, Dimethylsulfoxid, Dioxan, Propionitril oder Gemische untereinander.

Die Umsetzung wird mit einem Überschuss oder equimolarer Menge an dem entsprechenden Oxonium-Tetrafluoroborat, Sulfonium-Tetrafluoroborat oder Triphenylcarbonium-Tetrafluoroborat durchgeführt.

Die beschriebene Methode ist ebenfalls zur Einführung der Anionen [(Phenyl)₄B]⁻, PF₆⁻, SbF₆⁻ oder AsF₆⁻ in ionische Flüssigkeiten mit Onium-Kationen geeignet, indem Alkyloxonium-Salze oder Alkylsulfoniumsalze mit den entsprechenden Anionen mit Onium-Halogeniden umgesetzt werden.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

Es versteht sich für den Fachmann von selbst, dass in den vorab und nachfolgend genannten Verbindungen Substituenten wie beispielsweise H, N, O, Cl, F durch die entsprechenden Isotope ersetzt sein können.

Die NMR-Spektren wurden an Lösungen in deuterierten Lösungsmitteln bei 20°C an einem Bruker ARX 400 Spektrometer mit einem 5 mm Breitbandkopf ¹H/BB mit Deuterium Lock gemessen, falls nicht in den Beispielen angegeben. Die Messfrequenzen der verschiedenen Kerne sind: ¹H: 400,13 MHz und ¹⁹F: 376,50 MHz. Die Methode der Referenzierung wird bei jedem Spektrum bzw. bei jedem Datensatz separat angegeben.

### Beispiele:

### Beispiel 1: Synthese von 1-Hexyl-3-methylimidazolium-Tetrafluoroborat

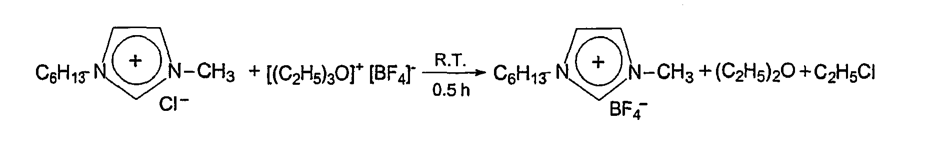

Zu 2.21 g (10.90 mmol) 1-Hexyl-3-methylimidazoliumchlorid werden 2.09 g (11.01 mmol) triethyloxonium Tetrafluoroborat zugegeben. Die Reaktionsmischung wird für 30 Minuten bei Raumtemperatur gerührt und anschließend alle flüchtigen Produkte im Vakuum von 13.3 Pa und 80°C (Temperatur des Ölbads) innerhalb von 30 Minuten entfernt. Man erhält 2.77 g 1-Hexyl-3-methylimidazolium Tetrafluoroborat als Flüssigkeit. Die Ausbeute ist annähernd quantitativ.

¹H NMR (Referenz: TMS; CD₃CN), ppm: 0.87 m (CH₃); 1.29 m (3CH₂); 1.81 m (CH₂); 3.82 s (CH₃); 4.11 t (CH₂); 7.34 d,d (CH); 7.38 d,d (CH); 8.47 br. s. (CH); ³J_{H,H} = 7.1 Hz; J_{H,H} = 1.8 Hz.
¹⁹F NMR (Referenz: CCl₃F-intern; CD₃CN), ppm: -150.2 (BF₄).

### Beispiel 2: Synthese von 1-Cyano-4-dimethylaminopyridinium-Tetrafluoroborat

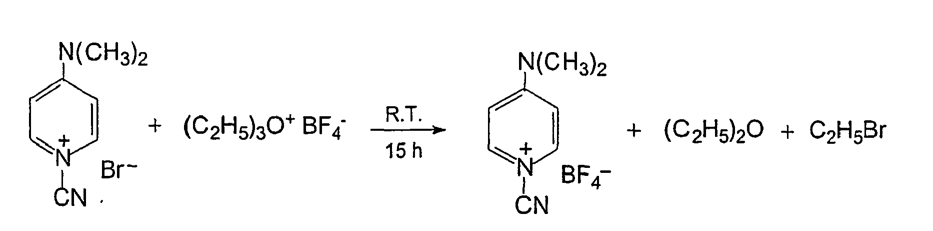

Zu einer Suspension von 2.13 g (9.34 mmol) 1-Cyano-4-dimethylaminopyridiniumbromid in 5 ml trockenem Dichlormethan werden 2.95 g (15.53 mmol) Triethyloxonium Tetrafluoroborat in 10 ml trockenem Dichlormethan zugegeben. Die Reaktionsmischung wird bei Raumtemperatur für 15 Stunden gerührt. Alle flüchtigen Produkte werden im Vakuum bei 13.3 Pa und Raumtemperatur während einer Stunde entfernt. Der Rückstand wird in 10 ml trockenem Acetonitril aufgenommen und 1-Cyano-4-dimethylaminopyridinium Tetrafluoroborat fällt bei einer Zugabe von 30 ml Ethylacetat aus. Der Niederschlag wird abfiltriert und im Vakuum bei Raumtemperatur getrocknet. Man erhält 1.40 g des Feststoffs. Bei partieller Destillation des Lösungsmittels fallen noch einmal 0.39 g an. Die Ausbeute an 1-Cyano-4-dimethylaminopyridinium Tetrafluoroborat beträgt daher zusammen 1.79 g, das entspricht 81.6 %.

¹H NMR (Referenz: TMS; CD₃CN), ppm 3.32 s (2CH₃); 6.98 d,m (2CH, A); 8.05 d,m (2CH, B); ³J_{H(A),H(B)} = 8.1 Hz.
¹⁹F NMR (Referenz: CCl₃F-intern; CD₃CN), ppm: -150,6 s (BF₄).
¹³C NMR (Referenz: TMS; CD₃CN), ppm: 42.2 q,q [N(CH₃)₂]; 107.6 m (CN); 109.8 d,m (2CH); 141.5 d,m (2CH); 158.0 m (C); ¹J_{C,H} = 195 Hz; ¹J_{C,H} = 175 Hz; ¹J_{C,H} = 142 Hz ; ³J_{C,H} = 3.3 Hz.

Raman-Spektrum: 2266.7 cm⁻¹ (CN).
Elementaranalyse C₈H₁₀BF₄N₃ (Mol. masse 234.99):
gefunden: C 40.78 %, H 4.57 %, N 18.10 %
berechnet: C 40.89 %, H 4.29 %, N 17.88%.

### Beispiel 3:

Analog zu Beispiel 1 werden
1-Methylimidazoliumchlorid mit Diethyloxonium-Tetrafluoroborat zu 1-Methylimidazolium-Tetrafluoroborat;
1-Butylimidazoliumchlorid mit Diethyloxonium-Tetrafluoroborat zu 1-Butylimidazolium-Tetrafluoroborat;
1-Ethyl-3-methyl-imidazoliumchlorid mit Diethyloxonium-Tetrafluoroborat zu 1-Ethyl-3-methylimidazolium-Tetrafluoroborat;
1-Butyl-3-methylimidazoliumchlorid mit Diethyloxonium-Tetrafluoroborat zu 1-Butyl-3-methylimidazolium-Tetrafluoroborat;
1-Methyl-3-pentylimidazoliumchlorid mit Diethyloxonium-Tetrafluoroborat zu 1-Methyl-3-pentylimidazolium-Tetrafluoroborat;
3-Methyl-1-octylimidazoliumchlorid mit Diethyloxonium-Tetrafluoroborat zu 3-Methyl-1-octylimidazolium-Tetrafluoroborat;
1-Decyl-3-methylimidazoliumchlorid mit Diethyloxonium-Tetrafluoroborat zu 1-Decyl-3-methylimidazolium-Tetrafluoroborat;
1-Dodecyl-3-methylimidazoliumchlorid mit Diethyloxonium-Tetrafluoroborat zu 1-Dodecyl-3-methylimidazolium-Tetrafluoroborat;
3-Methyl-1-tetradecylimidazoliurnchlorid mit Diethyloxonium-Tetrafluoroborat zu 3-Methyl-1-tetradecylimidazolium-Tetrafluoroborat;
1-Benzyl-3-methylimidazoliumchlorid mit Diethyloxonium-Tetrafluoroborat zu 1-Benzyl-3methylimidazolium-Tetrafluoroborat;
3-Methyl-1-phenylimidazoliumchlorid mit Diethyloxonium-Tetrafluoroborat zu 3-Methyl-1-phenylimidazolium-Tetrafluoroborat;
1-Ethyl-2,3-dimethylimidazoliumchlorid mit Diethyloxonium-Tetrafluoroborat zu 1-Ethyl-2,3-dimethylimidazolium-Tetrafluoroborat;
1-Butyl-2,3-dimethylimidazoliumchlorid mit Diethyloxonium-Tetrafluoroborat zu 1-Butyl-2,3-dimethylimidazolium-Tetrafluoroborat;
1-Hexyl-2,3-dimethylimidazoliumchlorid mit Diethyloxonium-Tetrafluoroborat zu 1-Hexyl-2,3-dimethylimidazolium-Tetrafluoroborat oder
1-Hexyldecyl-2,3-dimethylimidazoliumchlorid mit Diethyloxonium-Tetrafluoroborat zu 1-Hexyldecyl-2,3-dimethylimidazolium-Tetrafluoroborat umgesetzt.

### Beispiel 4: Synthese von 1-Butyl-pyridinium-Tetrafluoroborat

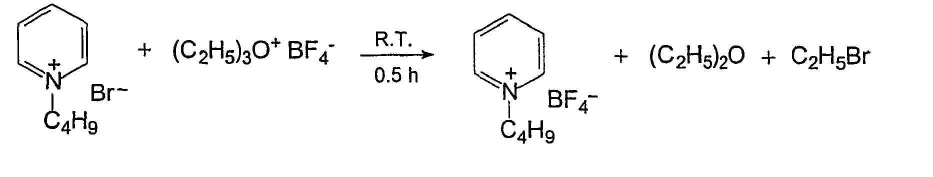

Zu einer Lösung von 2.77 g (12.82 mmol) 1-Butyl-pyridiniumbromid in 10 ml trockenem Dichlormethan werden 2.48 g (13.04 mmol) Triethyloxonium Tetrafluoroborat zugegeben. Die Reaktionsmischung wird für 30 Minuten bei Raumtemperatur gerührt. Anschließend werden alle flüchtigen Produkte im Vakuum von 13.3 Pa und 80°C (Ölbadtemperatur) innerhalb von 30 Minuten entfernt. Man erhält 2.82 g 1-Butyl-pyridinium Tetrafluoroborat als Flüssigkeit. Die Ausbeute ist annähernd quantitativ.

¹H NMR (Referenz: TMS; CD₃CN), ppm: 0.95 t (CH₃); 1.37 m (CH₂); 1.95 m (CH₂); 4.54 t (CH₂); 8.04 m (2CH); 8.52 t,t (CH); 8.73 d (2CH); ³J_{H,H} = 7.3 Hz; ³J_{H,H} = 7.6 Hz; ³J_{H,H} = 7.9 Hz; ³J_{H,H} = 5.7 Hz; ⁴J_{H,H} = 1.2 Hz.
¹⁹F NMR (Referenz: CCl₃F-intern; CD₃CN), ppm: -150,2 (BF₄).

### Analog dazu werden

1-Hexylpyridiniumchlorid mit Triethyloxonium-Tetrafluoroborat zu 1-Hexylpyridiniu m-Tetrafluoroborat;
1-Butyl-4-methylpyridiniumchlorid mit Triethyloxonium-Tetrafluoroborat zu 1-Butyl-4-methylpyridinium-Tetrafluoroborat;
1-Butyl-3-methylpyridiniumbromid mit Triethyloxonium-Tetrafluoroborat zu 1-Butyl-3-methylpyridinium-Tetrafluoroborat oder
1-Butyl-3-ethylpyridiniumbromid mit Triethyloxonium-Tetrafluoroborat zu 1-Butyl-3-ethylpyridinium-Tetrafluoroborat
umgesetzt.

### Beispiel 5: Synthese von 1-Ethyl-1-methylpyrrolidiniumTetrafluoroborat

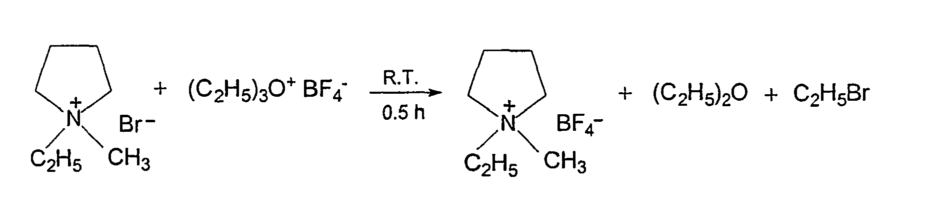

Zu einer Lösung von 2.45 g (12.62 mmol) 1-Ethyl-1-methyl-pyrrolidiniumbromid in 10 ml trockenem Dichlormethan werden 2.40 g (12.63 mmol) Triethyloxonium Tetrafluoroborat zugegeben. Die Reaktionsmischung wird für 30 Minuten bei Raumtemperatur gerührt. Anschließend werden alle flüchtigen Produkte im Vakuum von 13.3 Pa und 80°C (Ölbadtemperatur) innerhalb von 30 Minuten entfernt. Man erhält 2.53 g 1-Ethyl-1-methylpyrrolidinium Tetrafluoroborat. Die Ausbeute ist annähernd quantitativ.

¹H NMR (Referenz: TMS; CD₃CN), ppm: 1.31 t,m (CH₃); 2.13 m (2CH₂); 2.93 s (CH₃); 3.32 q (CH₂); 3.39 m (2CH₂); ³J_{H,H} = 7.3 Hz.
¹⁹F NMR (Referenz: CCl₃F-intern ; CD₃CN), ppm: -150.4 s (BF₄).

### Analog dazu werden

1-Butyl-1-methylpyrrolidinium-chlorid mit Triethyloxonium-Tetrafluoroborat zu 1-Butyl-1-methylpyrrolidinium-Tetrafluoroborat;
1-Hexyl-1-methylpyrrolidinium-chlorid mit Triethyloxonium-Tetrafluoroborat zu 1-Hexyl-1-methylpyrrolidinium-Tetrafluoroborat;
1-Methyl-1-octylpyrrolidinium-chlorid mit Triethyloxonium-Tetrafluoroborat zu 1-Methyl-1-octylpyrrolidinium-Tetrafluoroborat;
Trihexyl-tetradecylphosphoniumchlorid mit Triethyloxonium-Tetrafluoroborat zu
Trihexyl-tetradecylphosphonium-Tetrafluoroborat
umgesetzt.

### Beispiel 6: Synthese von N,N,N',N'-Tetramethyl-N"-ethylguanidinium-Tetrafluoroborat

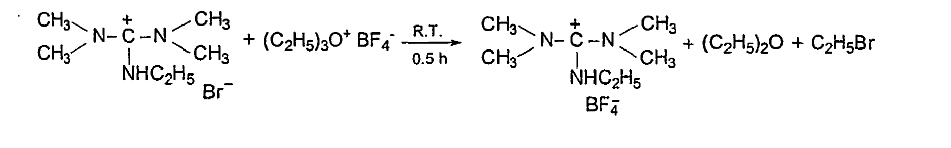

Zu einer Lösung von 3.73 g (16.64 mmol) N,N,N',N'-Tetramethyl-N"-ethylguanidiniumbromid in 10 ml trockenem Dichlormethan werden 3.20 g (16.83 mmol) Triethyloxonium Tetrafluoroborat zugegeben. Die Reaktionsmischung wird für 30 Minuten bei Raumtemperatur gerührt. Anschließend werden alle flüchtigen Produkte im Vakuum von 13.3 Pa und 80°C (Ölbadtemperatur) innerhalb von 30 Minuten entfernt. Man erhält 3.84 g N,N,N',N'-Tetramethyl-N"-ethylguanidinium Tetrafluoroborat. Die Ausbeute ist annähernd quantitativ.

¹H NMR (Referenz: TMS; CD₃CN), ppm: 1.11 t (CH₃); 2.86 br.s; 2.87 br.s; 2.91 s (4CH₃); 3.20 m (CH₂); 6.17 br.s (NH); ³J_{H,H} = 7.1 Hz.
¹⁹F NMR (Referenz: CCl₃F - intern; CD₃CN), ppm: -150.4 s (BF₄).

### Beispiel 7: Synthese von Tetrabutylphosphonium-Tetrafluoroborat

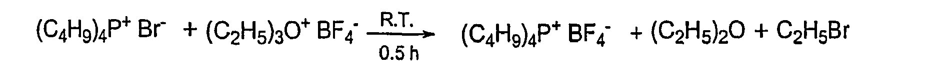

Zu einer Lösung von 3.81 g (11.23 mmol) Tetrabutylphosphoniumbromid in 10 ml trockenem Dichlormethan werden 2.14 g (11.27 mmol)

Triethyloxonium Tetrafluoroborat zugegeben. Die Reaktionsmischung wird für 30 Minuten bei Raumtemperatur gerührt. Anschließend werden alle flüchtigen Produkte im Vakuum von 13.3 Pa und 80°C (Ölbadtemperatur) innerhalb von 30 Minuten entfernt. Man erhält 3.88 g Tetrabutylphosphonium Tetrafluoroborat. Die Ausbeute ist annähernd quantitativ.

¹H NMR (Referenz: TMS; CD₃CN), ppm: 0.94 t (CH₃); 1.47 m (2CH₂); 2.05 m (CH₂); ³J_{H,H} = 7.1 Hz.
¹⁹F NMR (Referenz: CCl₃F - intern ; CD₃CN), ppm: -150.4 s (BF₄).

### Beispiel 8: Synthese von 1-Butyl-3-methylimidazoliumtetrafluoroborat

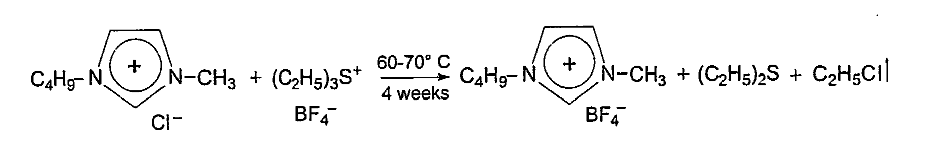

Zu 5.98 g (34.2 mmol) festem 1-Butyl-3-methylimidazoliumchlorid werden 7.06 g (34.3 mmol) Triethylsulfoniumtetrafluoroborat, (C₂H₅)₃S⁺ BF₄⁻, zugefügt. Die Reaktionsmischung wird bei 60-70°C (Temperatur des Ölbades) und unter Inertgasatmosphäre (Stickstoff) für einen Zeitraum von 4 Wochen gerührt. Alle flüchtigen Produkte werden bei einer Badtemperatur von 70°C und bei einem Druck von 13.3 Pa innerhalb von 3 Stunden abgepumpt. Man erhält 7.74 g einer Flüssigkeit. Die Ausbeute an 1-Butyl-3-methylimidazoliumtetrafluoroborat ist nahezu quantitativ. Das erhaltene Produkt wird mittels NMR-Spektroskopie untersucht.

¹H NMR (Referenz: TMS; Lösungsmittel: CD₃CN), ppm: 0.91 t (CH₃); 1.29 m (CH₂); 1.79 m (CH₂); 3.82 s (CH₃); 4.13 t (CH₂); 7.36 d,d (CH); 7.39 d,d (CH); 8.61 br. s. (CH); ³J_{H,H} = 7.2 Hz; J_{H,H} = 1.5 Hz.
¹⁹F NMR (Referenz: CCl₃F-intern; Lösungsmittel: CD₃CN), ppm: -150.1 (BF₄).

### Beispiel 9: Synthese von 1-Hexyl-3-methylimidazoliumtetrafluoroborat

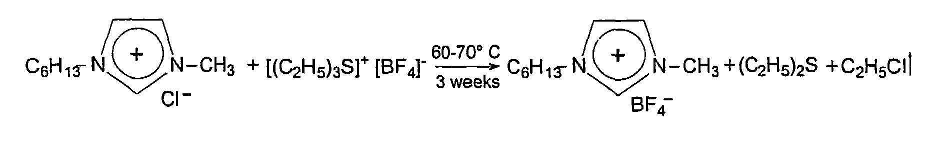

Zu 5.28 g (26 mmol) flüssigen 1-Hexyl-3-methylimidazoliumchlorids werden 5.38 g (26.1 mmol) Triethylsulfoniumtetrafluoroborat, (C₂H₅)₃S⁺ BF₄⁻, zugefügt. Die Reaktionsmischung wird bei 60-70°C (Temperatur des Ölbades) und unter Inertgasatmosphäre (Stickstoff) für einen Zeitraum von 3 Wochen gerührt. Alle flüchtigen Produkte werden bei einer Badtemperatur von 70°C und bei einem Druck von 13.3 Pa innerhalb von 3 Stunden abgepumpt. Man erhält 6.6 g einer Flüssigkeit. Die Ausbeute an 1-Hexyl-3-methylimidazoliumtetrafluoroborat ist nahezu quantitativ. Das erhaltene Produkt wird mittels NMR-Spektroskopie untersucht.

¹H NMR (Referenz: TMS; Lösungsmittel: CD₃CN), ppm: 0.87 m (CH₃); 1.29 m (3CH₂); 1.81 m (CH₂); 3.82 s (CH₃); 4.11 t (CH₂); 7.34 d,d (CH); 7.37 d,d (CH); 8.50 br. s. (CH); ³J_{H,H} = 7.1 Hz; J_{H,H} = 1.5 Hz.

¹⁹F NMR (Referenz: CCl₃F-intern; Lösungsmittel: CD₃CN), ppm: -150.2 (BF₄).

### Beispiel 10: Synthese von 1-Butyl-pyridiniumtetrafluoroborat

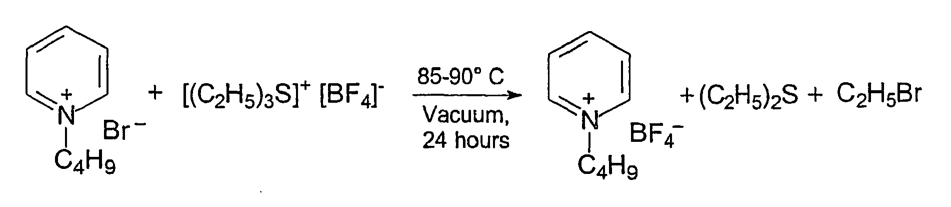

Eine Mischung aus 4.82 g (22.3 mmol) N-Buyl-pyridiniumbromid und 4.62 g (22.4 mmol) Triethylsulfoniumtetrafluoroborat, (C₂H₅)₃S⁺ BF₄⁻, werden bei 85-90°C (Temperatur des Ölbades) bei einem dynamischen Druck von 7 Pa innerhalb von 24 Stunden umgesetzt. Nach Abkühlung auf Raumtemperatur werden 4.97 g eines Öls erhalten. Die Ausbeute an N-Butyl-pyridiniumtetrafluoroborat ist nahezu quantitativ. Das erhaltene Produkt wird mittels NMR-Spektroskopie untersucht.

¹H NMR (Referenz: TMS; Lösungsmittel: CD₃CN), ppm: 0.93 t (CH₃); 1.35 m (CH₂); 1.95 m (CH₂); 4.58 t (CH₂); 8.05 m (2CH); 8.52 t,t (CH); 8.82 d (2CH); ³J_{H,H} = 7.6 Hz; ³J_{H,H} = 7.2 Hz; ³J_{H,H} = 7.9 Hz; ⁴J_{H,H} = 1.4 Hz.

¹⁹F NMR (Referenz: CCl₃F-intern; Lösungsmittel: CD₃CN), ppm: -150.1 (BF₄).

### Beispiel 11: Synthese von S-Ethyl-N,N,N',N'-tetramethylthiouroniumtetrafluoroborat

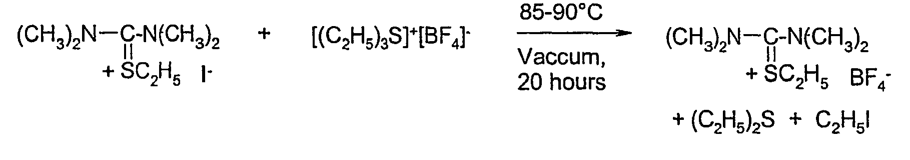

Eine Mischung aus 1.07 g (3.71 mmol) S-Ethyl-N,N,N',N'-tetramethylthiouroniumiodid und 0.77 g (3.74 mmol) Triethylsulfoniumtetrafluoroborat, (C₂H₅)₃S⁺ BF₄⁻, werden bei 85-90°C (Temperatur des Ölbades) bei einem dynamischen Druck von 7 Pa innerhalb von 20 Stunden umgesetzt. Nach Abkühlung auf Raumtemperatur werden 0.92 g eines Feststoffs erhalten. Die Ausbeute an S-Ethyl-N,N,N',N'-tetramethylthiouroniumtetrafluoroborat ist nahezu quantitativ. Der Schmelzpunkt beträgt 72-76°C. Das erhaltene Produkt wird mittels NMR-Spektroskopie untersucht.

¹H NMR (Referenz: TMS; Lösungsmittel: CD₃CN), ppm: 1.31 t (CH₃); 3.01 q (CH₂); 3.23 s (4CH₃); ³J_{H,H} = 7.4 Hz.

¹⁹F NMR (Referenz: CCl₃F-intern; Lösungsmittel: CD₃CN), ppm: -150.5 (BF₄).

### Beispiel 12: Synthese von 1-Hexyl-3-methylimidazoliumtetrafluoroborat_{.}

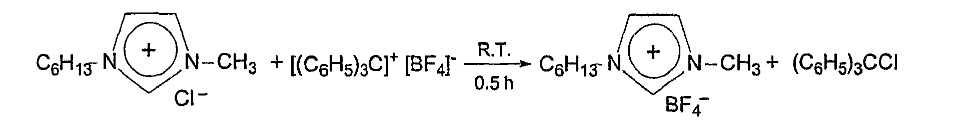

Zu 0.56 g (2.76 mmol) 1-Hexyl-3-methylimidazoliumchlorid werden 0.912 g (2.76 mmol) Triphenylcarboniumtetrafluoroborat, (C₆H₅)₃C⁺ BF₄⁻ und 5 cm³ Benzol zugegeben. Die Reaktionsmischung wird bei raumtemperatur für 30 Minuten gerührt. Die obere (Benzol)-Phase wird abgetrennt und das Produkt dreimal mit 10 ml Benzol gewaschen. Der Rückstand wird im Vakuum bei 13.3 Pa bei einer Badtemperatur von 100°C getrocknet. Es werden 0.7 g einer Flüssigkeit erhalten. Die Ausbeute an 1-Hexyl-3-methylimidazoliumtetrafluoroborat ist nahezu quantitativ. Das erhaltene Produkt wird mittels NMR-Spektroskopie untersucht.

¹H NMR (Referenz: TMS; Lösungsmittel: CD₃CN), ppm 0.89 m (CH₃); 1.31 m (3CH₂); 1.82 m (CH₂); 3.84 s (CH₃); 4.11 m (CH₂); 7.36 d,d (CH); 7.39 d,d (CH); 8.50 br. s. (CH); ³J_{H,H} = 7.2 Hz; J_{H,H} = 1.7 Hz.

¹⁹F NMR (Referenz: CCl₃F-intern; Lösungsmittel: CD₃CN), ppm: -150.2 (BF₄)

## Patentansprüche

1. Verfahren zur Herstellung von Onium-Tetrafluoroboraten der Formeln (5) bis (8)
[XR₄]⁺ [BF₄]⁻ (5)
[(R¹R²N)-C(=SR⁷)(NR³R⁴)]⁺ [BF₄]⁻ (6)
[C(NR¹R²N)(NR³R⁴)(NR⁵R⁶)]⁺ [BF₄]⁻ (7)
[HetN]⁺ [BF₄]⁻ (8),
durch Umsetzung eines Onium-Halogenids der Formel (1),
[XR₄]⁺ Hal⁻ (1),
wobei
X N, P
Hal Cl, Br oder I und
R jeweils unabhängig voneinander
H, wobei nicht alle Substituenten R gleichzeitig H sein dürfen, geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann bedeutet,
wobei ein oder mehrere R teilweise oder vollständig mit F substituiert sein können, wobei jedoch nicht alle vier oder drei R vollständig mit F substituiert sein dürfen,
und wobei ein oder zwei nicht benachbarte und nicht α- oder ω-ständige Kohlenstoffatome des R, durch Atome und/oder Atomgruppierungen ausgewählt aus der Gruppe -O-, -S-, -S(O)- oder-SO₂- ersetzt sein können
oder der Formel (2)
[(R¹R²N)-C(=SR⁷)(NR³R⁴)]⁺ Hal⁻ (2),
wobei
Hal Br oder I und
R¹ bis R⁷ jeweils unabhängig voneinander
Wasserstoff oder CN, wobei Wasserstoff für R⁷ ausgeschlossen wird, geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁷ teilweise oder vollständig mit F substituiert sein können, wobei jedoch nicht alle Substituenten an einem N-Atom vollständig mit F substituiert sein dürfen, wobei die Substituenten R¹ bis R⁷ paarweise durch Einfach- oder Doppelbindung miteinander verbunden sein können und wobei ein oder zwei nicht benachbarte, nicht direkt am Heteroatom gebundene und nicht ω-ständige Kohlenstoffatome der Substituenten R¹ bis R⁶ durch Atome und/oder Atomgruppierungen ausgewählt aus der Gruppe -O-, -S-, -S(O)- oder -SO₂- ersetzt sein können oder der Formel (3)
[C(NR¹R²)(NR³R⁴)(NR⁵R⁶)]⁺ Hal⁻ (3),
wobei,
Hal Cl, Br oder I und
R¹ bis R⁶ jeweils unabhängig voneinander
Wasserstoff oder CN,
geradkettiges oder verzweigtes Alkyl mit 1 bis 20 C-Atomen, geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
gesättigtes, teilweise oder vollständig ungesättigtes Cycloalkyl mit 3-7 C-Atomen, das mit Alkylgruppen mit 1-6 C-Atomen substituiert sein kann, bedeutet,
wobei ein oder mehrere der Substituenten R¹ bis R⁶ teilweise oder vollständig mit F substituiert sein können, wobei jedoch nicht alle
Substituenten an einem N-Atom vollständig mit F substituiert sein dürfen, wobei die Substituenten R¹ bis R⁶ paarweise durch Einfach- oder Doppelbindung miteinander verbunden sein können und wobei ein oder zwei nicht benachbarte, nicht direkt am Heteroatom gebundene und nicht ω-ständige Kohlenstoffatome der Substituenten R¹ bis R⁶ durch Atome und/oder Atomgruppierungen ausgewählt aus der Gruppe -O-, -S-, -S(O)- oder -SO₂- ersetzt sein können oder der Formel (4) entspricht,
[HetN]⁺ Hal⁻ (4)
wobei
Hal Cl, Br oder I und
HetN⁺ ein heterocyclisches Kation, ausgewählt aus der Gruppe 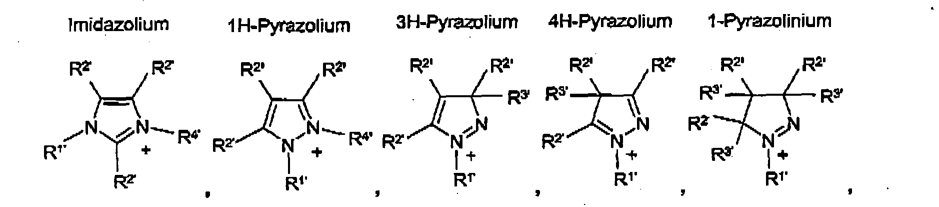 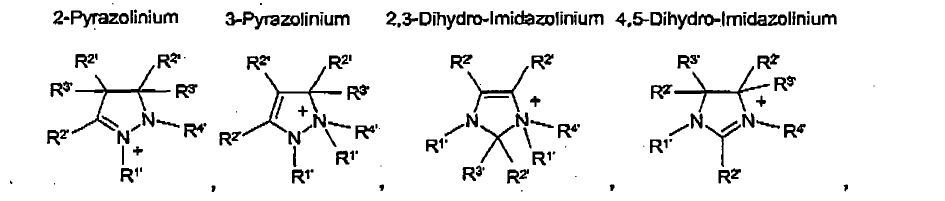 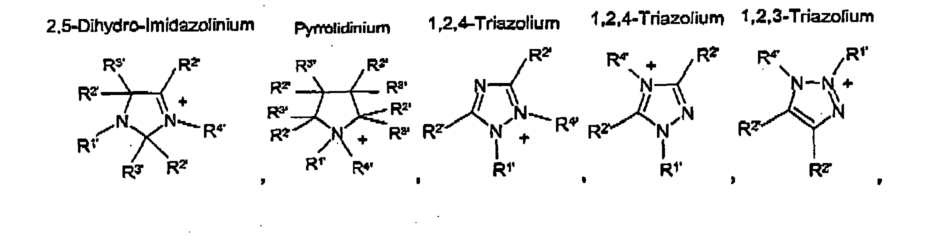 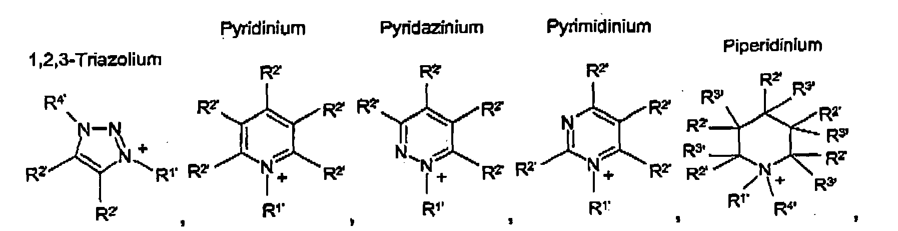 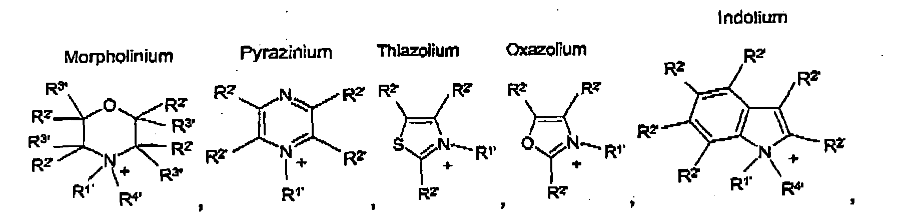 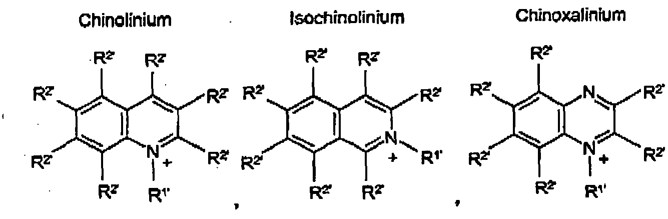 oder 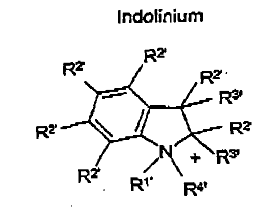 bedeutet, wobei die Substituenten
R¹' bis R⁴' jeweils unabhängig voneinander
Wasserstoff oder CN,
geradkettiges oder verzweigtes Alkyl mit 1-20 C-Atomen,
geradkettiges oder verzweigtes Alkenyl mit 2-20 C-Atomen und einer oder mehreren Doppelbindungen,
geradkettiges oder verzweigtes Alkinyl mit 2-20 C-Atomen und einer oder mehreren Dreifachbindungen,
Dialkylamino mit Alkylgruppen mit 1-4 C-Atomen, welches jedoch nicht am Heteroatom des Heterocyclus gebunden vorliegt,
gesättigtes, teilweise oder vollständig ungesättigtes Cycloalkyl mit 3-7 C-Atomen, das mit Alkylgruppen mit 1-6 C-Atomen substituiert sein kann oder Aryl-C₁-C₆-alkyl bedeutet,
wobei die Substituenten R¹' und R⁴' teilweise oder vollständig mit F substituiert sein können, wobei jedoch nicht gleichzeitig R¹' und R⁴' CN sind oder vollständig mit F substituiert sein dürfen,
wobei die Substituenten R²' und R³' teilweise oder vollständig mit Halogenen oder teilweise mit NO₂ oder CN substituiert sein dürfen und wobei ein oder zwei nicht benachbarte, nicht direkt am Heteroatom gebundene und nicht ω-ständige Kohlenstoffatome der Substituenten R¹' bis R⁴', durch Atome und/oder Atomgruppierungen ausgewählt aus der Gruppe -O-, -S-, -S(O)- oder -SO₂- ersetzt sein können,
mit einem Trialkyloxonium-Tetrafluoroborat, Trialkylsulfonium-Tetrafluoroborat oder Triphenylcarbonium-Tetrafluoroborat.

2. Verfahren nach Anspruch 1, **dadurch gekennzeichnet, dass** ein Oxonium-Tetrafluoroborat eingesetzt wird.

3. Verfahren nach Anspruch 1 oder 2, **dadurch gekennzeichnet, dass** die Reaktion bei Umsetzung mit Trialkyloxonium-Tetrafluoroborat oder Triphenylcarbonium-Tetrafluoroborat bei Temperaturen zwischen 0° und 100°C und bei Umsetzung mit Sulfonium-Tetrafluoroborat bei Temperaturen zwischen 0 und 150°Cdurchgeführt wird.

4. Verwendung der Verfahren nach einem der Ansprüche 1 bis 3 zur Reinigung von Onium-Tetrafluoroboraten, die durch Onium-Halogenide verunreinigt sind.

## Claims

1. Process for the preparation of onium tetrafluoroborates of the formulae (5) to (8)
[XR₄]⁺ [BF₄]⁻ (5)
[(R¹R²N)-C(=SR⁷)(NR³R⁴)]⁺ [BF₄]⁻ (6)
[C(NR¹R²N)(NR³R⁴)(NR⁵R⁶)]⁺ [BF₄]⁻ (7)
[HetN]⁺ [BF₄]⁻ (8),
by reaction of an onium halide of the formula (1)
[XR₄]⁺ Hal⁻ (1),
where
X denotes N, P
Hal denotes Cl, Br or I and
R in each case, independently of one another, denotes H, where all substituents R cannot simultaneously be H, straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more R may be partially or fully substituted by F, but where all four or three R must not be fully substituted by F,
and where, in the R, one or two non-adjacent carbon atoms which are not in the α- or ω-position may be replaced by atoms and/or atom groups selected from the group -O-, -S-, -S(O)- or -SO₂-,
or of the formula (2)
[(R¹R²N)-C(=SR⁷)(NR³R⁴)⁺ Hal⁻ (2),
where
Hal denotes Br or I and
R¹ to R⁷ each, independently of one another, denotes hydrogen or CN, where hydrogen is excluded for R⁷,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more of the substituents R¹ to R⁷ may be partially or fully substituted by F, but where all substituents on an N atom must not be fully substituted by F,
where the substituents R¹ to R⁷ may be connected to one another in pairs by a single or double bond
and where, in the substituents R¹ to R⁶, one or two non-adjacent carbon atoms which are not bonded directly to the heteroatom and are not in the ω-position may be replaced by atoms and/or atom groups selected from the group -O-, -S-, -S(O)- or -SO₂-,
or of the formula (3)
[C(NR¹R²)(NR³R⁴)(NR⁵R⁶)]⁺ Hal⁻ (3),
where
Hal denotes Cl, Br or I and
R¹ to R⁶ each, independently of one another, denotes hydrogen or CN,
straight-chain or branched alkyl having 1 to 20 C atoms,
straight-chain or branched alkenyl having 2-20 C atoms and one or more double bonds,
straight-chain or branched alkynyl having 2-20 C atoms and one or more triple bonds,
saturated, partially or fully unsaturated cycloalkyl having 3-7 C atoms, which may be substituted by alkyl groups having 1-6 C atoms,
where one or more of the substituents R¹ to R⁶ may be partially or fully substituted by F, but where all substituents on an N atom must not be fully substituted by F,
where the substituents R¹ to R⁶ may be connected to one another in pairs by a single or double bond
and where, in the substituents R¹ to R⁶, one or two non-adjacent carbon atoms which are not bonded directly to the heteroatom and are not in the ω-position may be replaced by atoms and/or atom groups selected from the group -O-, -S-, -S(O)- or -SO₂-,
or of the formula (4)
[HetN]⁺ Hal⁻ (4)
where
Hal denotes Cl, Br or I and
HetN⁺ denotes a heterocyclic cation selected from the group 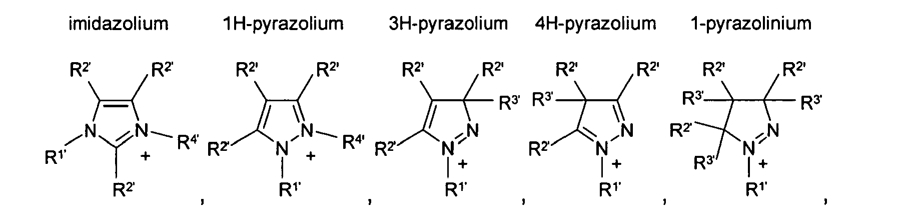 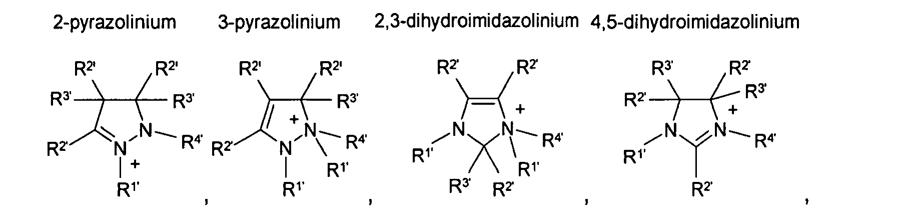 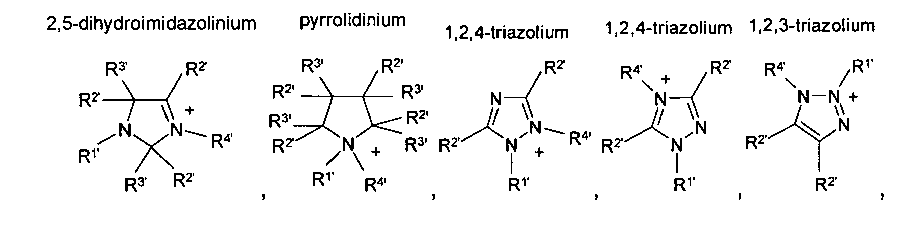 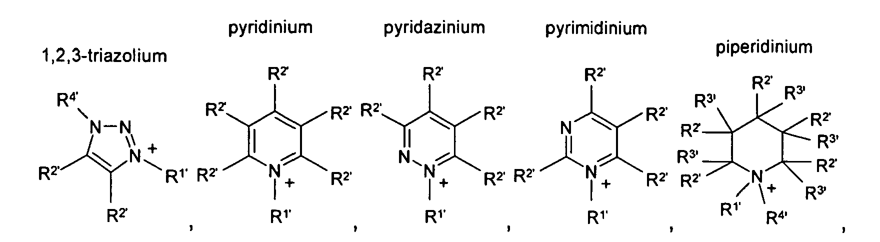 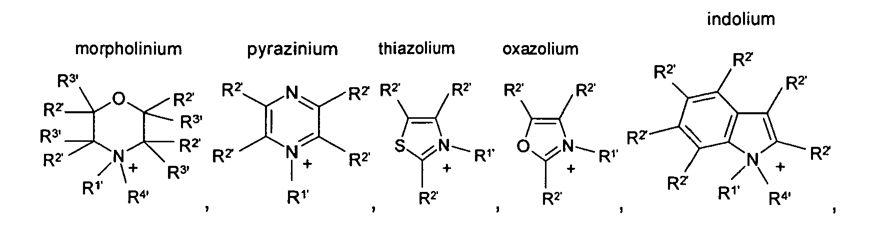 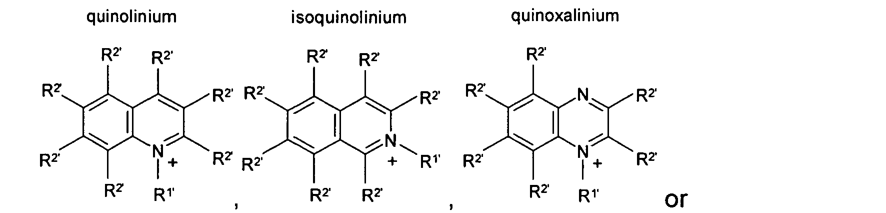 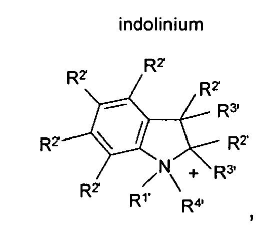 where the substituents
R¹' to R⁴' each, independently of one another, denotes hydrogen or CN,
straight-chain or branched alkyl having 1-20 C atoms,
straight-chain or branched alkenyl having 2-20 C atoms and one or more double bonds,
straight-chain or branched alkynyl having 2-20 C atoms and one or more triple bonds,
dialkylamino containing alkyl groups having 1-4 C atoms, but which is not bonded to the heteroatom of the heterocycle,
saturated, partially or fully unsaturated cycloalkyl having 3-7 C atoms, which may be substituted by alkyl groups having 1-6 C atoms,
or aryl-C₁-C₆-alkyl,
where the substituents R¹' and R⁴' may be partially or fully substituted by F, but where R¹' and R⁴' are not simultaneously CN or must not simultaneously be fully substituted by F,
where the substituents R²' and R³' may be partially or fully substituted by halogens or partially by NO₂ or CN
and where, in the substituents R¹' to R⁴', one or two non-adjacent carbon atoms which are not bonded directly to the heteroatom and are not in the ω-position may be replaced by atoms and/or atom groups selected from the group -O-, -S-, -S(O)- or -SO₂-,
with a trialkyloxonium tetrafluoroborate, trialkylsulfonium tetrafluoroborate or triphenylcarbonium tetrafluoroborate.

2. Process according to Claim 1, **characterised in that** an oxonium tetrafluoroborate is employed.

3. Process according to Claim 1 or 2, **characterised in that** the reaction is carried out at temperatures between 0° and 100°C in the case of reaction with trialkyloxonium tetrafluoroborate or triphenylcarbonium tetrafluoroborate and at temperatures between 0 and 150°C in the case of reaction with sulfonium tetrafluoroborate.

4. Use of the processes according to one of Claims 1 to 3 for the purification of onium tetrafluoroborates which are contaminated by onium halides.

## Revendications

1. Procédé de préparation de tétrafluoroborates d'onium de formules (5) à (8)
[XR₄]⁺ [BF₄]⁻ (5)
[(R¹R²N)-C(=SR⁷)(NR³R⁴)]⁺ [BF₄]⁻ (6)
[C(NR¹R²N)(NR³R⁴)(NR⁵R⁶)]⁻ [BF₄]⁻ (7)
[HétN]⁺ [BF₄]⁻ (8),
par réaction d'un halogénure d'onium de formule (1)
[XR₄]⁺ Hal⁻ (1),
où
X désigne N, P
Hal désigne CI, Br ou I et
R désigne dans chaque cas, indépendamment les uns des autres,
H, où tous les substituants R ne peuvent être simultanément H,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
où un ou plusieurs R peuvent être partiellement ou totalement substitués par F, mais où tous les quatre ou trois R ne doivent pas être totalement substitués par F,
et où, dans R, un ou deux atomes de carbone non adjacents qui ne sont pas en position α ou ω peuvent être remplacés par des atomes et/ou des groupes d'atomes choisis dans le groupe -O-, -S-, -S(O)- ou -SO₂-,
ou de formule (2)
[(R¹R²N)-C(=SR⁷)(NR³R⁴)]⁺ Hal⁻ (2),
où
Hal désigne Br ou I et
R¹ à R⁷ désignent chacun, indépendamment les uns des autres, hydrogène ou CN, où hydrogène est exclus pour R⁷,
alkyle à chaîne linéaire ou ramifiée ayant 1 à 20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
où un ou plusieurs parmi les substituants R¹ à R⁷ peuvent être partiellement ou totalement substitués par F, mais où tous les substituants sur un atome de N ne doivent pas être totalement substitués par F,
où les substituants R¹ à R⁷ peuvent être reliés l'un à l'autre par paires par une liaison simple ou double
et où, dans les substituants R¹ à R⁶, un ou deux atomes de carbone non adjacents qui ne sont pas liés directement à l'hétéroatome et ne sont pas en position ω peuvent être remplacés par des atomes et/ou des groupes d'atomes choisis dans le groupe -O-, -S-, -S(O)- ou -SO₂-,
ou de formule (3)
[C(NR¹R²)(NR³R⁴)(NR⁵R⁶)]⁺ Hal⁻ (3),
où
Hal désigne Cl, Br ou I et
R¹ à R⁶ désignent chacun, indépendamment les uns des autres, hydrogène ou CN,
alkyle à chaîne linéaire ou ramifiée ayant 1 à 20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
cycloalkyle saturé, partiellement ou totalement insaturé ayant 3-7 atomes de C, pouvant être substitué par des groupements alkyle ayant 1-6 atomes de C,
où un ou plusieurs parmi les substituants R¹ à R⁶ peuvent être partiellement ou totalement substitués par F, mais où tous les substituants sur un atome de N ne doivent pas être totalement substitués par F,
où les substituants R¹ à R⁶ peuvent être reliés l'un à l'autre par paires par une liaison simple ou double
et où, dans les substituants R¹ à R⁶, un ou deux atomes de carbone non adjacents qui ne sont pas liés directement à l'hétéroatome et ne sont pas en position ω peuvent être remplacés par des atomes et/ou des groupes d'atomes choisis dans le groupe -O-, -S-, -S(O)- ou -SO₂-,
ou de formule (4)
[HétN]⁺ Hal⁻ (4)
où
Hal désigne Cl, Br ou I et
HétN⁺ désigne un cation hétérocyclique choisi dans le groupe constitué par 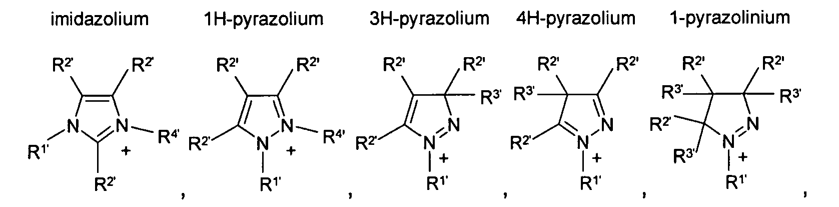 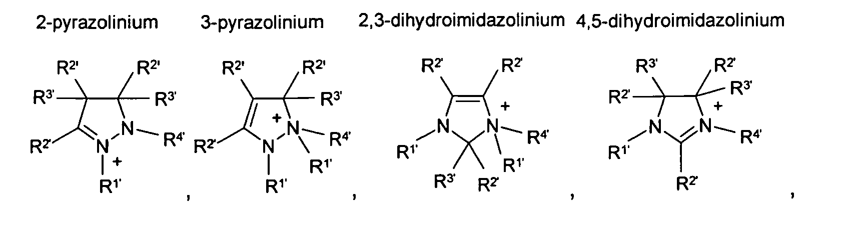 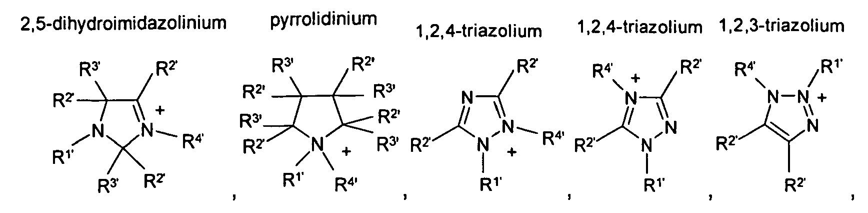 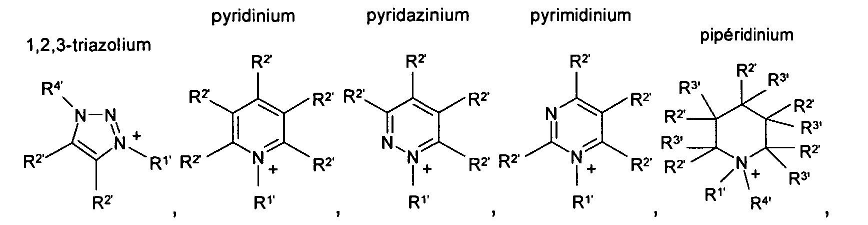 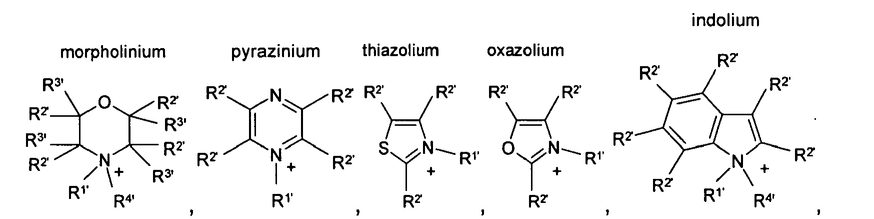 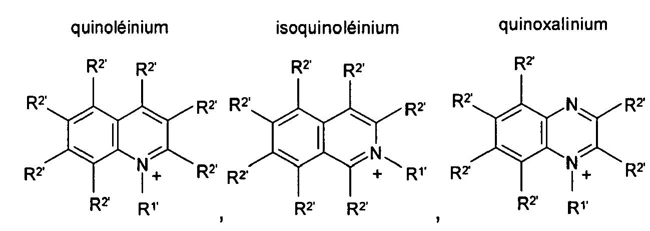 ou 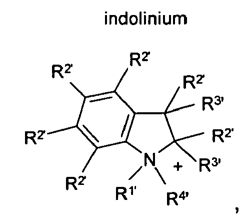 où les substituants
R¹' à R⁴' désignent chacun, indépendamment les uns des autres, hydrogène ou CN,
alkyle à chaîne linéaire ou ramifiée ayant 1-20 atomes de C,
alcényle à chaîne linéaire ou ramifiée ayant 2-20 atomes de C et une ou plusieurs doubles liaisons,
alcynyle à chaîne linéaire ou ramifiée ayant 2-20 atomes de C et une ou plusieurs triples liaisons,
dialkylamino contenant des groupements alkyle ayant 1-4 atomes de C, mais qui n'est pas lié à l'hétéroatome de l'hétérocycle,
cycloalkyle saturé, partiellement ou totalement insaturé ayant 3-7 atomes de C, pouvant être substitué par des groupements alkyle ayant 1-6 atomes de C,
ou aryl-C₁-C₆-alkyle,
où les substituants R¹' et R⁴' peuvent être partiellement ou totalement substitués par F, mais où R¹' et R⁴' ne sont pas simultanément CN ou ne doivent pas être simultanément totalement substitués par F,
où les substituants R²' et R³' peuvent être partiellement ou totalement substitués par des halogènes ou partiellement par NO₂ ou CN et où, dans les substituants R¹' à R⁴', un ou deux atomes de carbone non adjacents qui ne sont pas liés directement à l'hétéroatome et ne sont pas en position ω peuvent être remplacés par des atomes et/ou des groupes d'atomes choisis dans le groupe -O-, -S-, -S(O)- ou -SO₂-, avec un tétrafluoroborate de trialkyloxonium, un tétrafluoroborate de trialkylsulfonium ou un tétrafluoroborate de triphénylcarbonium.

2. Procédé selon la revendication 1, **caractérisé en ce qu'**on emploie un tétrafluoroborate d'oxonium.

3. Procédé selon la revendication 1 ou 2, **caractérisé en ce que** la réaction est effectuée à des températures comprises entre 0° et 100°C dans le cas d'une réaction avec le tétrafluoroborate de trialkyloxonium ou le tétrafluoroborate de triphénylcarbonium et à des températures comprises entre 0 et 150°C dans le cas d'une réaction avec le tétrafluoroborate de sulfonium.

4. Utilisation des procédés selon l'une des revendications 1 à 3, pour la purification de tétrafluoroborates d'onium qui sont contaminés par des halogénures d'onium.
